# EUROPEAN PATENT APPLICATION

(11) **EP 0 657 157 A1**
(43) Date of publication of application: **14.06.1995**
(21) Application number: 94308862.5
(22) Date of filing: 30.11.1994
(51) Int. Cl.: A61K 7/06

(54) **Styling aid containing a volatile alkylmethylsilicone**

(30) Priority: 06.12.1993 US 161367
(71) Applicant: DOW CORNING CORPORATION, Midland, Michigan 48686-0994 (US)
(72) Inventor: Vincent, Judith Mervane, Midland, Michigan (US); Hami, Annette Marie, Midland, Michigan (US)
(74) Representative: Vandamme, Luc Johan Roger

(57) **Abstract**

A hair styling aid containing a mixture of an alkyl alcohol solvent having more than one and less than five carbon atoms; a film forming copolymeric or terpolymeric resin; water; a volatile short chain linear or cyclic alkylmethylsilicone fluid; with the remainder of the mixture being materials such as neutralizers, perfumes and fragrances. The styling aid is characterized by being free of a plasticizer.

## Description

This invention is directed to a plasticizer free of styling aid, low volatile organic compounds (VOC) content, containing a volatile short chain linear or cyclic silicone fluid having in the molecule methyl and alkyl groups which include six, seven or eight carbon atoms.

Styling aids for hair are marketed in many types and categories and the simplest form of a styling aid is hairspray. These products are applied to dry hair after it has been arranged in a finished hairstyle. Hairsprays are designed to hold hair in place by a methodology which includes (i) a coating of each hair with a thin deposit of stiff polymer, and (ii) a gluing of the hairs together at points where they cross. Solvents contained in these products evaporate rapidly and the holding effect is instantaneous.

The main ingredients of hairspray are (i) a polymer which is a film forming resin; (ii) a solvent or solvent system; (iii) a plasticizer for the polymer; (iv) a neutralizer for the polymer; (v) a perfume; and (vi) adjuvants.

Early hairsprays relied upon shellac as the polymer, but this lacquer has now been replaced by modern synthetic film forming resinous materials. These materials include homopolymers, copolymers, terpolymers, quadripolymers and quaternized resins.

In actual practice, there is really only one solvent for non-aerosol hairsprays and that solvent is ethanol. Ethanol can be used alone or diluted with water. It possesses a rapid evaporation rate, an inoffensive odor which is easily masked by a perfume and excellent solvent properties for most resins. Its reasonable price is also attractive to formulators of consumer oriented product lines.

For example, the European Community has standardized throughout their common market, an ethanol which is denatured with denatonium benzoate and is marked with tert-butyl alcohol. The addition of water to the solvent system of a hairspray may be necessary, to improve the solubility of a hairspray ingredient which is not fully soluble in ethanol or to retard the evaporation rate in some specialty product lines. Under current United States environmental regulations, water can become an even more important part of the solvent package, since its inclusion falls under the category of non-VOC ingredients.

A plasticizer is used in a hairspray in a small quantity of about 0.5 percent by weight, to modify the properties of the film forming polymer resin, to make it more flexible. These materials include various types of liquid esters, proteins, polyols such as polypropylene glycol, lanolin derivatives, glycerine and silicone glycols.

A neutralizer as the name implies, neutralizes the film forming resin to an acceptable level for consumer application. Typically, neutralizers are commercially available amino alcohols such as 2-amino-2-methyl-1,3-propanediol (AMPD); 2-amino-2-ethyl-1,3-propanediol (AEPD); 2-amino-2-methyl-1-propanol (AMP-95®); 2-amino-1-butanol (AB); monoethanolamine (MEA); diethanolamine (DEA); triethanolamine (TEA); monoisopropanolamine (MIPA); diisopropanolamine (DIPA) and triisopropanolamine (TIPA).

Various types of perfumes and fragrances are used in hairsprays, to mask any unpleasant or offensive odor and to provide the consumer with a product which is more aesthetic and appealing to the senses. Among the adjuvants typically found in hairsprays, are small amounts of vitamins, proteins, amino acids and herbal extracts. If the hairspray is ethanol based, a preservative is not required. In aerosol products, a propellant is included such as carbon dioxide, nitrogen, nitrous oxide, butane, isobutane, propane, dichlorodifluoromethane, dichloro-tetrafluoroethane or dimethylether.

Setting lotions are styling aids much like hairsprays and differ only in that the setting lotion is applied to wet hair, whereas the hairspray is applied to dry hair. Water-soluble, film-forming resinous polymers can therefore be used but the alcohol content tends to be lower. Water based solvent packages can therefore be used for setting lotions, as the drying time is not critical as in the case of the hairspray.

Product shelves are filled with other types of styling aids and marketed under titles such as "spritz"; "spray gel"; and "sculpting spray". These products are typically only minor variants of one sort or another of a hairspray or a setting lotion. Styling aids, when mixed with a foaming agent and air, can be delivered in the form of a rather coarse foam which has a wet texture. This type of styling aid is marketed as a "mousse".

Almost any hair styling aid can enhance hair volume by virtue of a film-forming polymer resin being deposited on the individual hairs. This effect is even more pronounced in the case of styling aids which are applied to dry hair. In contrast, hair volume is reduced in the so-called "wet look", where sculpted hair styles tend to stick adjacent hairs together in parallel lines, rather than in a random orientation of the individual hairs which provides a more bulky appearance.

The feature which is unique about our invention, in comparison to the prior art, is that we have discovered that the particular volatile linear and cyclic alkylmethylsilicones of this invention, when substituted for a portion of the water in a low VOC compliant styling aid formulation, provide unexpected advantages and benefits to the styling aid.

Thus, these volatile linear or cyclic alkylmethyl-silicones provide to the styling aid an improved hold and better spray patterns. In addition, and quite unexpectedly, they (v) eliminate the need in styling aids for a separate plasticizer for the resin. Further, and even more unexpectedly, they (v) form compatible systems when combined with water and ethanol in certain prescribed ranges of these three ingredients. This latter feature (v) is particularly significant and unusual, when it is considered that typical solubility properties for volatile silicone fluids reveal that these fluids are usually insoluble in water. Yet, these same volatile silicone fluids are soluble in dehydrated ethanol of 99 percent purity when used at levels of parts of silicone to parts of solvent ranging from 10:1 to 1:10.

The invention relates to a styling aid which includes as an ingredient thereof, a volatile, short-chain linear or cyclic silicone fluid having in its molecule methyl groups and alkyl groups which may contain as many as twelve carbon atoms, but which preferably include six, seven or eight carbon atoms. Our styling aid is plasticized as a benefit of the inclusion of the silicone fluid and it meets the various regulations mandated by various environmental regulations, on the use of VOCs in consumer personal care products for consumers.

Thus, the styling aids of the present invention are formulated as aqueous ethanol solutions containing this silicone fluid. These aqueous solutions fully comply with known air pollution regulations.

Air pollution regulations limit the amount of organic solvents that can be discharged into the atmosphere. The term used for solvents is "volatile organic compounds" (VOC). A VOC is any compound of carbon that has a vapor pressure greater than 13.3 Pa (0.1 millimeter of mercury) at a temperature of 20°C. and a pressure of 101.3 kPa (760 millimeters mercury) or if the vapor pressure is unknown then any compound with less than twelve carbon atoms.

"Volatile organic content" has also been defined as the amount of VOCs liberated from a coating as determined by ASTM D3690 and EPA Reference Method 24, which are standard industrial tests. Under this definition, a VOC is any compound which enters the atmosphere and photochemically reacts in the atmosphere with nitrogen oxides to reduce ozone and form photochemical smog.

Reduction of VOC has been mandated in several states, and regulations in California, for example, require less than 180 grams of volatiles per liter of product to enter the atmosphere. This can be determined by baking ten grams of a product in an oven at 110°C. for one hour. The amount of solids which remain is subtracted from the total of the ten grams which was tested. Calculations are based on the weight of the volatiles that have evaporated, which is reported as grams per liter.

The United States Environmental Protection Agency (EPA) has identified many VOCs present in consumer products such as the common solvents of ethanol, isopropyl alcohol, kerosene and propylene glycol and hydrocarbon solvents such as isobutane, butane and propane (which are often employed as propellants in consumer products).

Some states, including the California Air Regulation Board (CARB), have standards which limit and reduce the amount of VOCs permitted in various consumer products, such as chemically formulated products used by household and institutional consumers. These regulations cover products such as detergents; cleaning compounds; polishes; floor products; cosmetics; personal care products; home, lawn and garden products; disinfectants; sanitizers and automotive specialty products.

These CARB type standards would effect such widely used consumer products as shaving lathers, hairsprays, shampoos, colognes, perfumes, aftershaves, deodorants, antiperspirants, suntan preparations, lotions, breath fresheners and room deodorants.

Thus, the need for new and novel formulations and techniques for reducing organic emissions are more than apparent.

The hair styling aid of our invention is a mixture of 50.0 to 95.0 percent by weight of an alkyl alcohol solvent which has more than one and less than five carbon atoms; 0.5 to 10.0 percent by weight of a film-forming copolymeric or terpolymeric resin; 1.0 to 15.0 percent by weight of water; and 1.0 to 45.0 percent by weight of a volatile, short-chain linear or cyclic alkylmethylsilicone fluid. Our hair styling aid may additionally contain other common adjuvants in small amounts such as a neutralizer, a perfume or a fragrance. The hair styling aid, however, is free of the separate plasticizer normally required by fixatives of the prior art.

The alkyl alcohol solvent is one of ethyl alcohol, propyl alcohol, isopropyl alcohol or tert-butyl alcohol. These alkyl alcohols are of particular utility in this invention, for the reason that they are known to be completely miscible with water. Ethyl alcohol is the most preferred alkyl alcohol solvent. In the examples, where reference is made to 200 proof ethanol, it is understood that this refers to pure 100 percent absolute dehydrated alcohol, as one-half of the proof number is the percentage of alcohol by volume.

The film-forming component of our hair styling aid is a copolymeric or terpolymeric resin. Suitable resins are polyvinylpyrrolidone ethyl methacrylate-methacrylic acid terpolymer; poly(vinylpyrrolidone-dimethylaminoethylmethacrylate) copolymer; vinyl acetate-crotonic acid copolymer; vinyl methyl ether-maleic anhydride copolymer; vinylacetate-crotonic acid-vinyl neodeconate terpolymer; poly(vinylpyrrolidone ethylmethacrylate) methacrylic acid copolymer; or derivatives of any of these resins. The resin of choice is the vinyl methyl ether-maleic anhydride copolymer, which is available commercially as a product and is sold under the trademark GANTREZ® ES225 by the GAF Chemicals Corporation, of Wayne, New Jersey USA.

The silicone component of our hair styling aid can be either a volatile, short-chain linear alkylmethylsilicone fluid or a volatile cyclic alkylmethylsilicone fluid.

The volatile, short-chain linear alkylmethyl-silicone fluid has the formula:

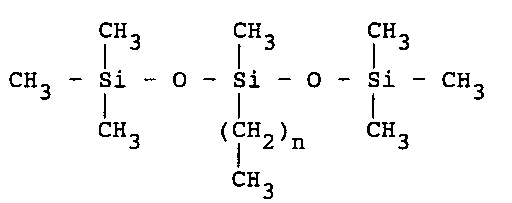

In the formula, the integer represented by n has a value of five to twelve. Preferably, n has a value of five to eight. Compounds most preferred are 3-hexylheptamethyltrisiloxane and 3-n-octyl-heptamethyltrisiloxane. Measured at 25°C., these preferred compounds have a viscosity of 2 mm²/s (centistokes) and 5 mm²/s (centistokes), respectively.

The volatile cyclic alkylmethylsilicone fluid has the following formula:

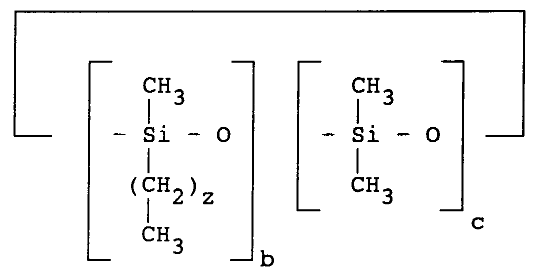

In this formula, the integer represented by z has a value of 1-12. The sum of b and c is four, five or six, with the proviso that b and c cannot each be zero. Examples of suitable cyclic compounds covered by this formula are instances wherein (i) b is one and c is three, four or five; (ii) b is two and c is two, three or four; (iii) b is three and c is one, two or three; (iv) b is four and c is one or two; and (v) b is five and c is one. The integer z is preferably five to eight.

Where other hair styling adjuvants are desired to be included, small amounts (generally less than about one percent by weight) may be added. For example, suitable among the various available neutralizers which can be used are any one or more of the amino alcohols such as 2-amino-2-methyl-1,3-propanediol (AMPD); 2-amino-2-ethyl-1,3-propanediol (AEPD); 2-amino-2-methyl-1-propanol (AMP-95®); 2-amino-1-butanol (AB); monoethanolamine (MEA); diethanolamine (DEA); triethanolamine (TEA); monoisopropanolamine (MIPA); diisopropanolamine (DIPA); and triisopropanolamine (TIPA). The neutralizer most preferred in this invention is 2-amino-2-methyl-1-propanol, which is a product manufactured and sold under the trademark AMP-95® by Angus Chemical Company, of Northbrook, Illinois USA.

Other common adjuvants which can be included are perfumes and fragrances to mask any unpleasant or offensive odor and vitamins, proteins, amino acids and herbal extracts. Ethanol based hair styling aids do not generally require a preservative. For aerosol delivery of the styling aid, a suitable propellant is included such as carbon dioxide, nitrogen, nitrous oxide, butane, isobutane, propane, dichlorodifluoromethane, dichlorotetrafluoroethane or dimethylether. The hair styling aids of this invention are also adapted to delivery to the hair in the form of a pump spray.

The following examples are set forth for the purpose of illustrating the hair styling aid and methods of using the styling aid in more detail. Amounts set forth in the examples in percent by weight refer to the percent by weight of each individual ingredient based on the total weight of the styling aid which is considered as one hundred. By incorporating into the styling aid 3-hexyl-heptamethyl-trisiloxane, as a non-VOC content ingredient, our examples show advantages of its use over water for that function.

### Example I

A "control" hair styling aid was prepared for use in evaluations against the styling aid of the present invention. The "control" was prepared by mixing together, in order, 10.0 percent by weight of GANTREZ® ES 225; 75.0 percent by weight of 200 proof ethanol; and 0.2 percent by weight of AMP-95®. To these ingredients, there was added 14.8 percent by weight of water. The water was added slowly and accompanied with stirring. GANTREZ® ES225 is a vinyl methyl ether-maleic anhydride copolymer and the material is available commercially as a fifty percent solution in ethanol made by GAF Chemicals Corporation, Wayne, New Jersey USA. AMP-95® is the material 2-amino-2-methyl-1-propanol, a commercially marketed product of Angus Chemical Company, Northbrook, Illinois USA.

### Example II

Hair styling aids II-VI according to the present invention are shown in Table I and were prepared by following the same procedure set forth in Example I, in which the same ingredients were employed. The difference between the "control" styling aid I and the styling aids II - VI of the invention, is that styling aids II - VI also included a percentage by weight of the volatile short chain linear alkylmethylsilicone fluid (VLAMS) 3-hexyl-heptamethyltrisiloxane which had a viscosity of two mm²/s (centistokes). The VLMAS was a replacement for an equal percentage of water in the formulation. The total of the VLMAS and water was 14.8 percent by weight in the styling aid. These styling aids are shown in Table I.

**TABLE I**

| Weight Percent in Styling Aid Formulation | | | | | |
|---|---|---|---|---|---|
| Styling Aid | Organic Resin | Neutraling Agent | Total Ethanol | Water | VLAMS |
| I | 5.0 | 0.2 | 80.0 | 14.8 | 0.0 |
| II | 5.0 | 0.2 | 80.0 | 12.8 | 2.0 |
| III | 5.0 | 0.2 | 80.0 | 10.8 | 4.0 |
| IV | 5.0 | 0.2 | 80.0 | 6.8 | 8.0 |
| V | 5.0 | 0.2 | 80.0 | 2.8 | 12.0 |
| VI | 5.0 | 0.2 | 80.0 | 0.0 | 14.8 |

Hair styling aids were evaluated by employing 15.2 cms (six inch) hair tresses of approximately two grams of untreated human hair. Each tress was made by gluing the top part of the hair to a 5.1 cms x 5.1 cms (2'' x 2'') plastic tab. After drying on the tab, the hair was trimmed to 15.2 cms (six inches). Each tress was then cleaned with an anionic/amphoteric shampoo of the following formulation:

| | |
|---|---|
| Distilled Water | 61.45% |
| Methylchlorisothiazolinone and methylisothiazolinone | 0.05% |
| Ammonium Lauryl Sulfate | 35.00% |
| Cocamide DEA | 3.00% |
| Citric Acid | q.s. |
| Ammonium Chloride | 0.50% |

The tress was first rinsed for 15 minutes under 40°C. tap water and 0.5 cm³ of the above shampoo was applied. Shampooing for 30 seconds was followed by a 30 second rinse. The tresses were then set on plastic rollers approximately 1.3 cms (1/2 inch) in diameter and allowed to dry overnight. Hair styling aids were applied to the hair either by dripping on 0.5 grams or by spraying on 0.3 grams. If the drip application was used, the hair was combed three times and reset on a roller. If the resin solution was delivered from a pump, the hair was not reset. The solution was allowed to cure on the hair for a period of one to two hours for ethanol-based and overnight for water-based formulations. The dried tresses were hung in a constant humidity chamber at 90 percent relative humidity and initial readings were taken as well as additional readings at predetermined intervals. If the tress was reset, the roller was removed prior to exposure. Percent curl was calculated as the extended length minus the length at the end of the predetermined interval divided by the extended length minus the initial length. The results are shown in Table II and represent the initial curl drop after two hours of exposure. Initial curl drop is an important factor in evaluating eighty percent VOC formulations.

Table II indicates that after two hours the initial curl drop was improved with a partial replacement of the water with the volatile linear alkylmethylsilicone. The initial curl drop was slightly affected with a total or nearly total, replacement of the water with the volatile linear alkylmethylsilicone. Percent curl was calculated as factor (15.3 - final length) times one hundred, divided by factor (15.3 - initial length). The percent curl of all tresses to start was one hundred percent.

**TABLE II**

| Initial Curl Drop Study at 90% RH, 80°F Average Percent Curl | | | |
|---|---|---|---|
| Styling Aid | 45 min. | 90 min. | 2 hr. |
| I | 92.4 | 87.6 | 83.8 |
| II | 97.1 | 96.2 | 91.4 |
| III | 93.2 | 90.4 | 86.6 |
| IV | 95.2 | 92.4 | 88.6 |
| V | 88.4 | 84.6 | 79.6 |
| VI | 91.2 | 85.4 | 75.6 |

Subjective evaluations of the six styling aids during the curl drop study revealed that hair treated with the "control" styling aid I was difficult to comb through and the hair had a rough feel. A slight flaking of the "control" styling aid resin on the hair was also noted. In contrast, hair treated with styling aids II through VI exhibited no resin flaking. The comb through improved as the amount of the alkylmethylsilicone increased in the formulation. It was also observed that the hair treated with the alkylmethylsilicone containing formulation had a softer feel than the control. Hair treated with formulations IV, V and VI exhibited a conditioned feel.

It was observed that decreasing the amount of water while increasing the amount of the volatile, linear alkylmethylsilicone in the styling aid improved the spray pattern. Styling aids I, III, IV and VI in Table I were dispensed by a Calmar Mark IV™ pump at a distance of 25.4 cms (10 inches) on a vertically mounted sheet of solvent sensitive paper. The spray pattern was allowed to dry in the vertical position. Dripping was observed with the control. The amount of dripping was decreased by increasing the amount of volatile linear alkylmethylsilicone substituted for the water in the formulation. No dripping was observed with styling aid VI, the formulation containing 14.8% alkylmethylsilicone and no water. The higher alkylmethylsilicone containing formulations produced a more evenly distributed spray pattern with finer droplets.

The alkylmethylsilicones of this invention can be produced by the reaction of a linear siloxane having Si-H functionality in the chain such as (Me₃SiO_{1/2})₂(OSiMeH)ₓ in which Me is methyl and x is forty to one hundred and a cyclic siloxane having (Me₂SiO) units of the formula (Me₂SiO)ₓ in which Me is methyl and x is an integer of three to six, preferably four or five. The reaction product is contacted with a slight stoichiometric excess of an alkene CH₂=CHR in the presence of a platinum on carbon catalyst and an alkylmethylsiloxane having the structure shown above is produced.

The alkylmethylsilicones of this invention can also be produced by the direct hydrolysis of methylhydrogen dichlorosilane to form cyclomethylhydrogen polysiloxanes or by the direct cohydrolysis of methylhydrogen dichlorosilane and dimethyl dichlorosilane to form cyclomethylhydrogensiloxydimethylsiloxy copolymers. The reaction product is contacted with a slight stoichiometric excess of an alkene CH₂=CHR in the presence of a platinum on carbon catalyst and an alkylmethylsiloxane having the structure shown above is produced.

Batch production of the alkylmethylsilicones is conducted by adding the reaction product to a non-agitated suspension of the catalyst in the alkene at 60°C. Continuous production of the alkylmethyl polysiloxanes is conducted by pumping a preheated solution of a five percent stoichiometric excess of an alkene CH₂=CHR and the reaction product through a packed column containing platinum on carbon catalyst chips. The column will require provision for the removal of heat because of the exothermic nature of the reaction.

The materials are further processed in accordance with the present invention in order to provide a more cosmetically acceptable product by removing from the product any remaining cyclic siloxane and any residual methylhydrogendimethylsiloxane cocyclics present as (MeHSiO)(Me₂SiO)₃. The alkylmethylsilicones produced by our invention have been found to contain at most 0.5 percent residual alkene and about 99.5 percent alkylmethylsilicone product. No measurable residual amount of platinum has been detected. The products are otherwise colorless, odorless, clear and stable materials.

Other variations and modifications may be made in the compounds, compositions and methods, described herein without departing from the essential features and concepts of the present invention.

## Claims

1. A hair styling aid comprising a mixture of (i) from 50.0 to 95.0 percent by weight of an alkyl alcohol solvent having more than one and less than five carbon atoms; (ii) from 0.5 to 10.0 percent by weight of a film forming resin selected from copolymeric or terpolymeric resins; (iii) from 1.0 to 15.0 percent by weight of water; (iv) from 1.0 to 45.0 percent by weight of a volatile silicone selected from short-chain linear alkylmethyl-silicone fluids or cyclic alkylmethylsilicone fluids; the remainder of the mixture being selected from neutralizers, perfumes and fragrances; the styling aid being characterized by being free of a plasticizer.

2. The hair styling aid according to claim 1 in which the alkyl alcohol solvent is selected from ethyl alcohol, propyl alcohol, isopropyl alcohol and tert-butyl alcohol.

3. The hair styling aid according to claim 1 in which the film forming copolymeric or terpolymeric resin is selected from polyvinylpyrrolidone ethyl methacrylate-methacrylic acid terpolymer; poly(vinylpyrrolidone-dimethylaminoethylmethacrylate) copolymer; vinyl acetate-crotonic acid copolymer; vinyl methyl ether-maleic anhydride copolymer; vinylacetate-crotonic acid-vinyl neodeconate terpolymer; poly(vinylpyrrolidone ethylmethacrylate) methacrylic acid copolymer and derivatives thereof.

4. The hair styling aid according to claim 1 in which the volatile, short-chain linear alkylmethylsilicone fluid is a compound having the formula: 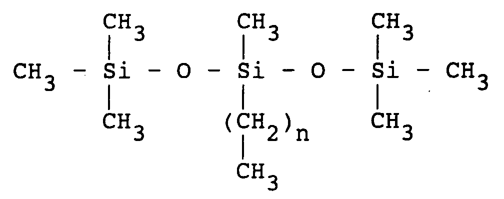 in which n is an integer having a value of five to twelve.

5. The hair styling aid according to claim 4 in which the volatile, short-chain linear alkylmethylsilicone fluid is a compound selected from 3-hexylheptamethyltrisiloxane or 3-n-octyl-heptamethyltrisiloxane.

6. The hair styling aid according to claim 1 in which the volatile, cyclic alkylmethylsilicone fluid is a compound having the formula: 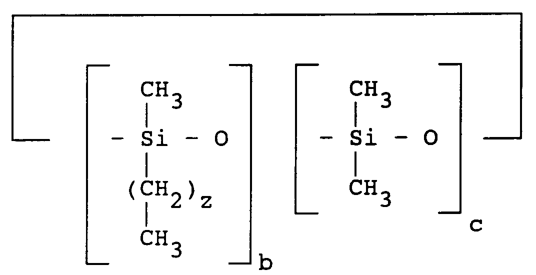 in which z has a value of 1-12; and the sum of b and c is four, five or six, with the proviso that b and c cannot be zero.

7. A method of holding hair in a style comprising applying to hair the hair styling aid of claim 1.
